(19) Europäisches Patentamt / European Patent Office / Office européen des brevets

(11) **EP 4 215 202 B1**

(12) **EUROPEAN PATENT SPECIFICATION**

(45) Date of publication and mention
of the grant of the patent:
**04.03.2026 Bulletin 2026/10**

(21) Application number: **22425001.9**

(22) Date of filing: **25.01.2022**

(51) International Patent Classification (IPC):
*A61K 35/744* (2015.01)    *A61K 35/747* (2015.01)
*A61K 41/10* (2020.01)    *A61P 15/02* (2006.01)
*A61K 9/00* (2006.01)    *A61K 9/48* (2006.01)
*A61K 47/36* (2006.01)

(52) Cooperative Patent Classification (CPC):
(C-Sets available)
A61P 15/02; A61K 9/0034; A61K 35/744;
A61K 35/747; A61K 47/36; A61K 9/4866    (Cont.)

(54) **COMPOSITION COMPRISING TINDALIZED BACTERIA FOR THE TREATMENT OF VAGINAL MUCOSA**

ZUSAMMENSETZUNG MIT TINDALISIERTEN BAKTERIEN ZUR BEHANDLUNG VON VAGINALSCHLEIMHAUT

COMPOSITION COMPRENANT DES BACTÉRIES TINDALISÉES POUR UNE UTILISATION DANS LA MUQUEUSE VAGINALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**26.07.2023 Bulletin 2023/30**

(73) Proprietor: **BIOFARMA S.R.L.**
**33036 Mereto di Tomba (UD) (IT)**

(72) Inventors:
• **Castorina, Sebastiano Maurizio**
**Segrate (MI) (IT)**
• **Vanelli, Arianna**
**Gallarate (VA) (IT)**
• **Murzilli, Stefania**
**Gallarate (VA) (IT)**
• **Taddei, Alessandro**
**Dairago (MI) (IT)**
• **Sabino, Fabiana**
**Palazzo San Gervasio (PZ) (IT)**

(74) Representative: **Petraz, Gilberto Luigi et al**
**GLP S.r.l.**
**Viale Europa Unita, 171**
**33100 Udine (IT)**

(56) References cited:
CN-A- 110 123 978    CN-A- 113 633 666
CN-A- 113 662 998    IT-A1- PD20 090 021

• GUARALDI CLAUDIA ET AL: "Tyndallized lactic ferments: new possible therapies in treating vaginitis", vol. 69, no. 1, 1 February 2017 (2017-02-01), XP055913583, ISSN: 2724-606X, Retrieved from the Internet <URL:http://dx.doi.org/10.23736/S0026-4784.16.03942-3> DOI: 10.23736/S0026-4784.16.03942-3
• DATABASE GNPD [online] MINTEL; 23 October 2018 (2018-10-23), ANONYMOUS: "Pure Glow Food Supplement", XP055914719, retrieved from https://www.gnpd.com/sinatra/recordpage/6081265/ Database accession no. 6081265
• N◊RIA PIQU◊; ET AL: "Health Benefits of Heat-Killed (Tyndallized) Probiotics: An Overview", INTERNATIONAL JOURNAL OF MOLECULAR SCIENCES, vol. 20, no. 10, 23 May 2019 (2019-05-23), pages 2534, XP055619578, DOI: 10.3390/ijms20102534
• L. LOPETUSO: "Gelatin tannate and tyndallized probiotics: a novel approach for treatment of diarrhea", EUROPEAN REVIEW FOR MEDICAL AND PHARMACOLOGICAL SCIENCES, vol. 21, 1 January 2017 (2017-01-01), pages 873 - 883, XP055531769

Remarks:
   The file contains technical information submitted
   after the application was filed and not included in this
   specification

(52) Cooperative Patent Classification (CPC): (Cont.)

   C-Sets
   **A61K 35/744, A61K 2300/00;**
   **A61K 35/747, A61K 2300/00**

Remarks:
   The file contains technical information submitted
   after the application was filed and not included in this
   specification

**Description**

FIELD OF THE INVENTION

Embodiments described here concern a composition for the treatment of the vaginal mucosa and a formulation comprising inactivated microorganisms and hyaluronic acid which comprises the composition.

BACKGROUND OF THE INVENTION

The vaginal ecosystem is made up of a variety of bacteria and metabolic products of the microbes and the host that coexist in a delicate balance. There are acids, carbohydrates, proteins and nucleic acids, fatty acids and sugars deriving from degrading bacteria. There are non-pathogenic and pathogenic microbial species that are present in a ratio of about 200: 1.

The balance is maintained by specific species of Lactobacillus and by the substances they produce which inhibit the growth of pathogenic bacteria. Imbalance in the ecosystem causes non-pathogenic and pathogenic bacteria to predominate over Lactobacillus and increases the risk of bacterial vaginitis (BV) 1. It can be difficult to determine the cause of the infection because Gram-positives and Gram-negatives, as well as aerobic and anaerobic bacteria, can also be found in a healthy vagina.

When appropriate environmental changes occur inside the vagina, the number of lactobacilli decreases, other bacteria become dominant, and an alteration of the vaginal microflora occurs. The alteration of the microflora and the vaginal ecosystem can lead the patient to become symptomatic.

In order to treat these conditions due to the imbalance of the microflora and the vaginal ecosystem, there are medical devices based on probiotics to be applied to the vaginal mucosa.

For instance, Guaraldi et al., 2017, Tyndallized lactic ferments: new possible therapies in treating vaginitis, Minerva Obstetrics and Gynecology, 69:1 discloses the use of a composition comprising a tyndallized mixture of bacteria (among which Lactobacillus casei and Streptococcus thermophilus, at an unspecified content) to improve healing and prevent recurrence of Candida vaginitis in pregnant women.

In addition, CN113662998 discloses a medicinal composition for treating vaginitis and gynecological inflammation including 8-12 parts by weight of Lactobacillus casei.

Finally, GNPD Mintel Pure Glow Food Supplement, database accession 6081265, discloses an encapsuled composition for oral use as a dietary supplement including 3% by weight of tyndallized Lactobacillus casei and 3% by weight of tyndallized Streptococcus thermophilus.

Probiotics are defined as live microorganisms which, if administered in adequate quantities, confer a health benefit to the host. Probiotics have been shown to be effective in the prevention and treatment of various pathological conditions.

One disadvantage of probiotics is connected to the relative scarcity of information on their mechanisms of action and on the existence of potentially safer alternatives that can provide similar benefits. One question that is addressed in several studies is whether probiotic bacteria have to be "alive" and able to proliferate and survive for prolonged periods in order to exert their beneficial effect. The lack of more information raises concerns about the potentially harmful effects of these agents.

Currently, the use of probiotics is contextualized within in strategies to prevent antimicrobial resistances. Indeed, in the current context of high levels of antibiotic resistance, the acquisition and transfer of resistance genes should be addressed in the assessment of the safety live probiotics and should be considered in the development of future products.

Another disadvantage of the use of probiotics is that their effectiveness, linked to their bacterial load, is limited over time due to the progressive death of the bacteria themselves. It should be noted that these bacteria are also sensitive to parameters such as temperature and humidity, which affect the duration of the effectiveness of probiotics.

Furthermore, probiotics also make it necessary to use "prebiotics" to support their attachment to the vaginal mucosa, as well as technological processes to make them "gastro-resistant". Prebiotics are non-digestible substances of food origin, generally water-soluble fibers, which, taken in adequate quantities, selectively promote the growth and activity of one or more bacteria already present in the body or taken together with the prebiotic.

Another disadvantage of probiotic bacteria is that they require considerable efforts for conservation, in particular the maintenance of a cold chain during all stages of production, storage, sale and consumption. Shelf life can also be a limitation for products that use probiotics.

There is therefore a need to perfect a composition for the treatment of the vaginal mucosa and a corresponding medical device which can overcome at least one of the disadvantages of the state of the art.

To do this, it is necessary to solve the technical problem of creating a composition for the treatment of the vaginal mucosa which is easier to manage and has a more lasting effect over time.

In particular, one purpose of the present invention is to provide a composition for the treatment of the vaginal mucosa which does not have a decay of the bacterial load of the product over time and which is not sensitive to parameters such as temperature and humidity.

Another purpose of the present invention is to provide a composition for the treatment of the vaginal mucosa which does not require the use of prebiotics or to provide technological processes of gastro-resistance for its formulation.

Another purpose is to provide a composition for the treatment of the vaginal mucosa that has an effective action in re-establishing the balance of the vaginal microflora and ecosystem.

The Applicant has devised, tested and embodied the present invention to overcome the shortcomings of the state of the art and to obtain these and other purposes and advantages.

SUMMARY OF THE INVENTION

The present invention is set forth and characterized in the independent claims. The dependent claims describe other characteristics of the present invention or variants to the main inventive idea.

In accordance with the above purposes and to resolve the technical problem described above in a new and original way, also obtaining considerable advantages compared to the previous state of the art, there is provided a composition for the treatment of the vaginal mucosa comprising inactivated microorganisms as a substitute for probiotics. In particular, the inactivated microorganisms are tindalized microorganisms, that is, microorganisms that have undergone a specific heat treatment able to render said microorganisms unable to metabolize and reproduce, since they are not alive.

In accordance with the invention, the composition for the treatment of the vaginal mucosa as above comprises tindalized *Lactobacillus casei* and tindalized *Streptococcus thermophilus,* as well as hyaluronic acid.

These two specific strains are chosen because of their natural presence in the unaltered vaginal ecosystem. Preferably, the tindalized microorganisms consist of tindalized *Lactobacillus casei* and tindalized *Streptococcus thermophilus.*

Tindalized *Lactobacillus casei* and tindalized *Streptococcus thermophilus* are each present at a concentration, by weight of the total weight of the composition, between 5% and 15%.

In some embodiments, the two microorganisms as above can be present in equal concentrations, or in different concentrations. The weight ratio between the two microorganisms *L. casei:S. thermophilus* can be comprised between 10:1 and 1:10, preferably between 5:1 and 1:5, more preferably between 2:1 and 1:2. An example can be a 1:1 ratio.

Preferably, the hyaluronic acid is present between 4% and 14% by weight of the weight of the composition, more preferably between 7% and 11%, even more preferably between 7.5% and 10%.

According to some embodiments described here, the composition also comprises inulin, preferably between 35% and 70%, more preferably between 45% and 65%, even more preferably between 50% and 60% by weight of the weight of the composition.

In other possible embodiments, the method also provides to associate corn starch, for example between 2% and 12% by weight of the total weight of the composition, preferably between 5% and 10%, more preferably between 6.5% and 8%.

In accordance with some embodiments, the composition can also comprise excipients and/or additives such as, for example, lubricants and/or desiccants. In particular, the composition can comprise corn starch and/or magnesium stearate.

The composition of the present invention can comprise, in addition or as a substitution, other additives and/or excipients of pharmaceutical or food grade, that is, substances without therapeutic activity suitable for pharmaceutical or food use. In the context of the present invention, the ingredients acceptable for pharmaceutical or food use comprise all the auxiliary substances known to the person of skill in the art such as, as a non-limiting example, diluents, solvents, solubilizers, thickeners, sweeteners, flavorings, dyes, lubricants, surfactants, antimicrobials, antioxidants, preservatives, buffers to stabilize the pH and mixtures thereof.

In some embodiments, the composition disclosed here is for vaginal use, as a medicament. In one embodiment, the composition according to the invention is for use in a method for the treatment, preventive and/or curative, of a pathology, symptom and/or disorder associated with dysfunctions of the vaginal mucosa in a subject in need.

According to another aspect of the present invention, a formulation is provided comprising a composition in accordance with the present description. This formulation is in particular provided for the treatment of the vaginal mucosa. In the formulation, tindalized *Lactobacillus casei* is present at a concentration between 5% and 15% by weight of the total weight of the formulation, and tindalized *Streptococcus thermophilus* is present at a concentration between 5% and 15% by weight of the total weight of the formulation.

The formulation can be, as a non-limiting example, in hard capsule, softgel, liquid, as a solution, biphasic liquid system, suspension, semi-solid form, such as gel, cream or foam, or solid form, such as powder, granules, tablets and equivalent forms. Preferably, the composition of the invention can be formulated in a formulation for vaginal use in capsule.

Other embodiments concern a method for the preparation of a formulation for the treatment of the vaginal mucosa. The method comprises associating tindalized *Lactobacillus casei* between 5% and 15% by weight of the total weight of the composition, and tindalized *Streptococcus thermophilus* between 5% and 15% by weight of the total weight of the composition and hyaluronic acid.

In possible embodiments, hyaluronic acid, is provided between 4% and 14% by weight of the total weight of the composition.

In other possible embodiments, the method also provides to associate inulin, for example between 35% and 70% by weight of the total weight of the composition.

In still other possible embodiments, the method also provides to associate corn starch, for example between 2% and 12% by weight of the total weight of the composition.

Finally, the present invention concerns a pharmaceutical or cosmetic composition, a vaginal lavage or a composition for a medical device comprising the composition of the present invention.

BRIEF DESCRIPTION OF THE DRAWINGS

These and other aspects, characteristics and advantages of the present invention will become apparent from the following description of some embodiments, given as a non-restrictive example with reference to the attached drawings wherein:

- fig. 1 is a histogram showing the evolution of the quantity of interleukin IL-1$\alpha$, as an indicator of the level of irritation, on a reconstructed three-dimensional tissue model of human vaginal epithelium in the presence and absence of composition for the treatment of the vaginal mucosa according to the invention; and
- fig. 2 is a histogram showing the evolution of tissue vitality on a reconstructed three-dimensional tissue model of human vaginal epithelium in the presence and absence of composition for the treatment of the vaginal mucosa according to the invention.

DESCRIPTION OF SOME EMBODIMENTS

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

All measurements are carried out, unless otherwise indicated, at 25°C (ambient temperature) and at atmospheric pressure. All temperatures, unless otherwise indicated, are expressed in degrees Celsius.

All percentages and ratios indicated shall be understood to refer to the weight of the total composition (w/w), unless otherwise indicated.

All percentage intervals reported here are provided with the provision that the sum with respect to the overall composition is 100%, unless otherwise indicated.

All the intervals reported here shall be understood to include the extremes, including those that report an interval "between" two values, unless otherwise indicated.

The present description also includes the intervals that derive from uniting or overlapping two or more intervals described, unless otherwise indicated.

The present description also includes the intervals that can derive from the combination of two or more values taken at different points, unless otherwise indicated.

By water, we mean distilled water, unless otherwise specified.

According to the present invention, a composition for the treatment of the vaginal mucosa comprises inactivated microorganisms, that is, in a form that is no longer alive. More precisely, the composition comprises tindalized microorganisms.

In particular, the microorganisms of the composition described here include tindalized *Lactobacillus casei* and tindalized *Streptococcus thermophilus.* In some embodiments described here, the tindalized microorganisms consist of tindalized *Lactobacillus casei* and tindalized *Streptococcus thermophilus;* however, in other embodiments there can be provided more than two species of tindalized microorganisms, even different to *Lactobacillus casei* or *Streptococcus thermophilus.*

*Streptococcus thermophilus* is a Gram-positive bacterium belonging to the *phylum Firmicutes, Streptococcaceae* family. It belongs to the class of lactic bacteria which comprise the species of the genera *Carnobacterium, Enterococcus, Lactobacillus, Lactococcus* and others. One of the most significant health benefits associated with the use of *S. thermophilus* in humans is the capacity of the bacterium to exert a positive effect on the levels of ceramide (a protective agent of the skin) in the body. *In vitro,* it has shown a significant positive impact on ceramide levels measured in cultured human keratinocytes which have a function in forming a barrier against environmental damage such as pathogens, heat, UV and water loss. *In vivo,* it has shown an equally beneficial effect on the level of ceramides in the stratum corneum, which forms a barrier to protect the underlying tissue from infection, dehydration, exposure to chemical substances and mechanical stress.

*Lactobacillus casei* (LCG), which is part of the *Lactobacillus casei* group together with *Lactobacillus paracasei* and *Lactobacillus rhamnosus,* is one of the most widely studied and applied probiotic species of lactobacilli, particularly for health promotion in the prevention of a number of diseases and disturbances. Health benefits associated with LCG have been reported for a variety of health conditions, ranging from atopic dermatitis to cancer. The mechanisms through which LCG directly or indirectly has a beneficial effect on human health are not yet fully understood and require further study. Potential mechanisms include the production of antimicrobial substances such as bacteriocins (for live bacteria), improving the epithelial barrier through attachment and competition for pathogenic binding sites.

In general, tindalized microorganisms are microorganisms that have been subjected to a specific heat treatment that has inactivated them, that is, it has made them unable to metabolize and reproduce. The activity of tindalized microorganisms is therefore not attributable to the possibility of generating new progeny and colonizing. Therefore, where the UFC/g unit is used, reference is made to the starting concentration of the culture which is then subjected to tindalization.

The tindalized microorganisms are heat treated and killed. The treatment is performed together with the culture medium, which is constantly controlled and optimized, which also contains the substances produced by the metabolism of the treated microorganisms. Thus, a product is obtained, advantageously in a lyophilized form, which contains both the probiotic cell parts and also the substances of the normal metabolism of the microorganisms, in particular vitamins, glycoproteins and micronutrients. The Applicant has found that the tindalized microorganisms can act as a barrier to the engraftment of pathogens, thus allowing the recolonization of the bacterial flora present.

It has been shown that the contribution of tindalized microorganisms provides a basis on which the vaginal microflora and ecosystem are reconstituted and there is a contrast to the establishment and development of all pathogenic or harmful microorganisms.

In addition, the tindalized microorganisms used in the composition described here adhere to the vaginal epithelial cells and create an effective barrier that limits pathological microbial colonization in the area of application, obtaining optimized conditions for the natural regeneration of tissues.

Their physiological effect, therefore, hinders the establishment of pathogenic germs (barrier effect), stimulates general well-being and facilitates the establishment of a beneficial bacterial flora.

The tindalization process provides a heat treatment of suspensions of microorganisms at a temperature range between 70 and 100 °C, and in some cases the inactivation is obtained with the combination of heat treatments with incubation periods at lower temperatures (ambient temperatures, cooling or freezing temperatures).

The tindalized microorganisms are each present between 5% and 15%. The weight ratio between the two microorganisms mentioned above is preferably 1:1, but it can be different according to each case, for example between 10:1 and 1:10, preferably between 5:1 and 1:5, more preferably between 2:1 and 1:2.

The composition also comprises hyaluronic acid, for example in the form of sodium hyaluronate. This compound, together with the specific tindalized microorganisms mentioned above, allows to obtain an effective barrier effect to protect the vaginal mucosa and also allows to lubricate and soothe, which favors the natural mechanisms of restoration of the physiological vaginal environment as well as the integrity, elasticity and resistance of the mucous membranes.

In fact, hyaluronic acid is a very hydrophilic compound and has muco-mimetic, mucus adhesive and viscoelastic properties that allow it to adhere well to the vaginal mucosa, increasing permanence time, hydrating and protecting the tissues.

In some embodiments, the hyaluronic acid can preferably be present between 4% and 14% by weight of the weight of the composition, more preferably between 7% and 11%, even more preferably between 7.5% and 10%.

The hyaluronic acid can be of any molecular weight whatsoever, that is, of low molecular weight, generally corresponding to 50-600 kDa, of medium molecular weight, generally corresponding to 600-1800 kDa, or of high molecular weight, generally corresponding to a molecular weight greater than 1800 kDa, in particular 1800-2200 kDa. Preferably, the hyaluronic acid can have an average molecular weight, more preferably between 1000 and 1800 kDa.

The average molecular weight is calculated on the basis of the intrinsic viscosity of the hyaluronic acid, measured using the method described in the European Pharmacopoeia (Ph. Eur.) relating to sodium hyaluronate, monograph no. 1472, 1/2009. The molecular mass is calculated by means of the formula

$$MW = ([\eta] \times 10^5/36)^{1/0.78}$$

where $[\eta]$ is the intrinsic viscosity of the hyaluronic acid measured as indicated above, expressed in $m^3/kg$.

In some embodiments, the composition described here can also comprise inulin. Although inulin is generally known for its prebiotic function, it has been observed that its use in the present composition, which comprises tindalized microorganisms, has a synergistic effect with such tindalized microorganisms, possibly also in the presence of the hyaluronic acid, which brings some surprising advantages in terms of coating and adhesion to the vaginal mucosa.

In some embodiments, the inulin can be preferably provided between 35% and 70%, more preferably between 45% and 65%, even more preferably between 50% and 60% by weight of the weight of the composition.

Advantageously, in some embodiments, the composition can also comprise corn starch as a thickener. The corn starch also allows to absorb part of the humidity that could prevent the formation of a coating film, or alter its properties, making it less effective. Corn starch can be provided between 2% and 12%, preferably between 5% and 10%, more preferably between 6.5% and 8% by weight of the weight of the composition.

The composition can also comprise silica dioxide as an anti-platelet agent. Like corn starch, silica dioxide also allows to absorb part of the humidity which could prevent the formation of a coating film or alter its properties. The silica dioxide can be present at a concentration comprised between 0.1% and 5%, preferably between 0.25% and 2.5%, even more preferably between 0.5% and 2% by weight of the total weight of the composition.

In some embodiments, the composition can also comprise magnesium stearate as a lubricating agent, advantageously between 0.1% and 5%, more advantageously between 0.25% and 2.5%, even more advantageously between 0.5% and 2% by weight of the weight of the composition. Preferably, the concentration of magnesium stearate can be substantially equal to the concentration of silica dioxide, if provided.

Favorably, the composition described above is for use in the treatment of the vaginal mucosa. Its mechanism of action provides the formation, thanks to the hydrating, emollient and filming properties of the tindalized microorganisms, of a protective barrier that adheres to the vaginal mucosa. This protective barrier impairs the engraftment and proliferation of pathogenic microorganisms and, at the same time, has a protective, hydrating and soothing action on the vaginal mucosa that facilitates the restoration of the vaginal physiological conditions, in particular in terms of integrity, elasticity and resistance, and promotes the resolution of typical symptoms associated with non-specific vaginosis and vaginitis.

The present invention also concerns a formulation, in particular for vaginal use, comprising the composition for the treatment of the vaginal mucosa described above, wherein tindalized *Lactobacillus casei* is present at a concentration between 5% and 15% by weight of the total weight of the formulation, and tindalized *Streptococcus thermophilus* is present at a concentration between 5% and 15% by weight of the total weight of the formulation.

This formulation is obtained by formulating the composition described above in forms such as to be applicable to the vaginal mucosa, for example in hard capsule, softgel, liquid, as solution, biphasic liquid system, suspension, semi-solid form, as gel, cream or foam, or solid form, such as powder, granules, tablets and equivalent forms.

Preferably, the formulation for vaginal use comprises a capsule in which the composition is enclosed. Such capsule is favorably made of a pharmacologically acceptable material which dissolves when subjected to the vaginal physiological conditions.

EXAMPLE

To study the barrier effect of the tested product made according to the present description, we assessed its capacity to reduce the effects of a substance with known irritating capacity (lipopolysaccharide, LPS) on reconstructed human vaginal epithelium (HVE). For this purpose, 3 HVE tissues were stimulated with LPS and 3 were stimulated with LPS in the presence of the tested product. 3 untreated HVE tissues (kept in PBS during the incubation period) were used as negative control. 4 contact times were considered: 30, 60, 120 and 180 minutes. The levels of interleukin-1$\alpha$ (IL-1$\alpha$) produced and the vitality of the tissues were measured by means of dye 3-(4,5-Dimethylthiazol-2-yl)-2,5-Diphenyltetrazolium Bromide, Thiazolyl Blue Tetrazolium Bromide (MTT) at each time.

The levels of IL-1$\alpha$ in the tissues stimulated with LPS and treated with the tested product are significantly lower than those of the tissues only stimulated with LPS after 30, 60 and 120 minutes of contact. Similarly, the vitality of the treated and stimulated tissues is greater than that of the tissues only stimulated and not treated after 30, 60 and 120 minutes of contact. It is therefore possible to recognize a barrier effect that remains at least up to 120 minutes of contact.

Skin irritation induced by chemical substances, which manifests itself mainly with erythema and edema, is the result of a cascade of events that begins with the penetration of the substances themselves through the stratum corneum and subsequent damage to the layers of keratinocytes and other underlying skin cells. The damaged cells can also release mediators of inflammatory processes and activate an inflammatory cascade that can also involve the cells of the dermis, in particular the cells of the stroma and the vascular endothelium. It is precisely the dilation and the increased permeability of blood vessels that are the main causes of erythema and edema. The HVE model, even in the absence of a vascularization system, allows to study the initial events of this cascade, such as for example the damage to cells and tissues, by analyzing cell vitality.

Cell vitality is measured by means of the enzymatic conversion of the MTT dye to a purple formazan salt which is quantified by means of spectrophotometry after extraction from the tissues. The irritant substances are recognized thanks to their ability to reduce cell vitality below well-defined threshold values.

### Materials and methods

### Tissues

Three-dimensional reconstructed vaginal epithelium tissue is an epithelium obtained from cells derived from a vaginal epidermoid carcinoma cultured on an inert polycarbonate filter at the liquid/air interface in a specific medium. This model forms an epithelial tissue without a stratum corneum, histologically similar to the vaginal epithelium in vivo.

### Tested substances

Negative control: phosphate buffer solution (PBS)
Positive control: Lipopolysaccharide (LPS)

The tested sample has the following composition:

| Ingredient | Quantity (mg per capsule) | Concentration (% in weight) |
|---|---|---|
| Tindalized *Streptococcus thermophilus* | 33.330 | 9.389 |
| Tindalized *L. casei* | 33.330 | 9.389 |
| Dried corn starch | 25.610 | 7.214 |
| Magnesium stearate | 3.550 | 1.000 |
| Silica dioxide | 3.550 | 1.000 |
| Sodium hyaluronate (Hyaluronic acid) | 33.410 (30.000) | 9.411 (8.451) |
| Soluble powder based on Inulin tit. 90% (Inulin) | 222.220 (200.000) | 62.597 (56.337) |

### Determination of cell vitality by means of MTT test

Cell vitality was measured by means of the MTT standard colorimetric assay for measuring the activity of enzymes that reduce MTT to formazan. The chemical basis of the test is, therefore, the reduction of MTT, a yellow substance in solution, to form purple formazan crystals. This reduction process occurs mainly in the cytoplasm, and to a lesser extent in the mitochondria and on the cell membrane, and is highly dependent on the intracellular concentrations of NADH and NADPH. As a result of these metabolic processes, within a few hours purple formazan crystals appear which can be dissolved in isopropanol. The absorption of the solubilized crystals can be measured at the wavelength of 540 nm and it is proportional to the number of live cells in a very wide linear range.

### Dosage of cytokines

The content of interleukins in the culture medium was determined by beans of ELISA (Enzyme-Linked Immunosorbent Assay) test, a very widespread biochemical technique used for the quantification of proteins and peptides. In an ELISA assay, an antigen specific to the interleukin to be identified is immobilized on a solid surface (the bottom of a well) to which the dosage medium is added. Detection is performed by means of a biotinylated secondary antibody and then reacted with streptavidin-HRP. The colorimetric reaction is proportional to the quantity of cytokine present. The results are read by means of a spectrophotometer at 450 nm. The values obtained were then interpolated into a standard interleukin curve.

### Experimental protocol

### Pre-incubation

HVE tissues were left in growth medium for at least 2 hours (37°C, 5% $CO_2$) before treatment.

### Treatment

HVE tissues were treated for 30, 60, 120 and 180 minutes with 16 $\mu$l of substance according to the following scheme:

- Negative control     PBS
- Positive control     LPS
- Treatment     Tested substance (TS) + LPS

Each experiment was conducted in triplicate.

### Washings

HVE tissues were repeatedly washed with PBS, in order to eliminate all traces of the substances used for the treatments.

### Post-incubation

HVE tissues were transferred to growth medium for 42 hours $\pm$ 60 minutes (37°C, 5% $CO_2$). At the end of this period, the mediums were recovered for the subsequent dosage of interleukins.

### MTT assay

HVE tissues were treated with MTT (1 mg/ml) for 3 hours (37°C, 5% $CO_2$) and then the formazan crystals were extracted in isopropanol for 2 hours at ambient temperature. The reading of the optical density was performed at 540 nm.

### Results

### Dosage of interleukins

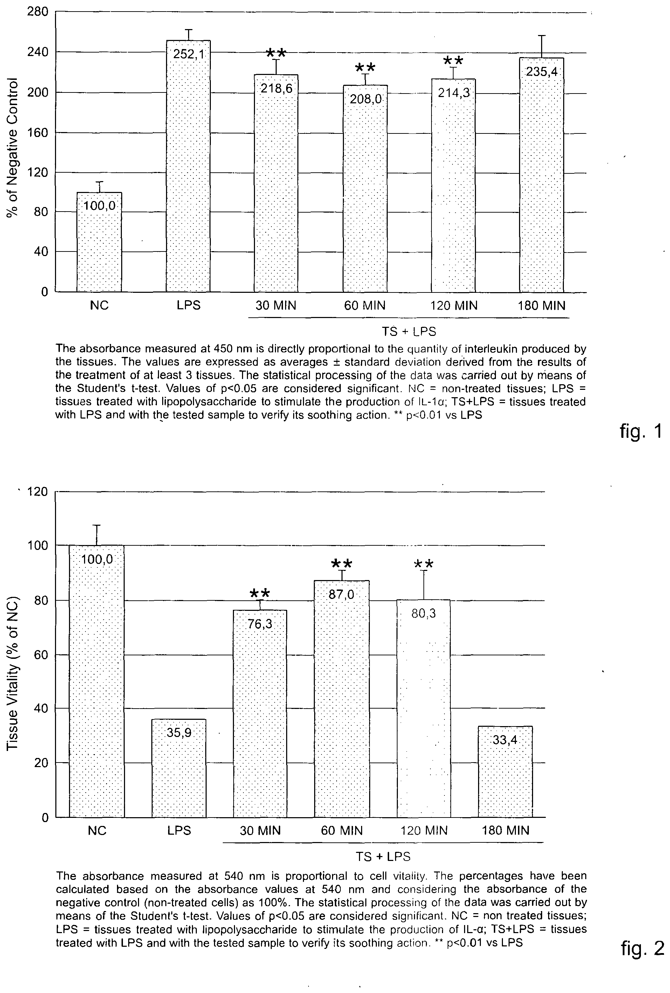

The result of the interleukin dosage is shown in the histogram of fig. 1. The tested product is able to significantly reduce the production of IL-1$\alpha$ after 30, 60 and 120 minutes of contact.

### Assessment of tissue vitality

The result of the assessment of tissue vitality is shown in the histogram of fig. 2. It can be deduced that in HVE tissues treated with the tested product, the vitality is significantly higher than that of the untreated tissues after 30, 60 and 120 minutes of contact.

### Conclusions

The tests carried out have allowed to show that the tested composition made according to the present invention has a protective barrier effect on the reconstructed human vaginal epithelium and that this effect is manifested up to at least 120 minutes of contact.

It is clear that modifications and/or additions of parts or steps may be made to the composition for the treatment of the vaginal mucosa and to the formulation as described heretofore, without departing from the field and scope of the present invention as defined by the claims.

It is also clear that, although the present invention has been described with reference to some specific examples, a person of skill in the art shall certainly be able to achieve many other equivalent forms of composition for the treatment of the vaginal mucosa and of medical device, having the characteristics as set forth in the claims and hence all coming within the field of protection defined thereby.

**Claims**

1. Composition for the treatment of the vaginal mucosa, **characterized in that it** comprises tindalized *Lactobacillus casei* between 5% and 15% by weight of the total weight of the composition, and tindalized *Streptococcus*

*thermophilus* between 5% and 15% by weight of the total weight of the composition, **and in that** it comprises hyaluronic acid.

2. Composition as in claim 1, **characterized in that** the hyaluronic acid is present between 4% and 14% by weight of the total weight of the composition.

3. Composition as in claim 1 or 2, **characterized in that** the hyaluronic acid has a molecular weight between 50 and 1800kDa, said molecular weight being measured by means of the formula MW = $([\eta] \times 10^5/36)^{1/0.78}$, in which $[\eta]$ is the intrinsic viscosity of the hyaluronic acid in $m^3/kg$ measured by means of the method defined in the European Pharmacopoeia (Ph. Eur.), monograph n. 1472, 1/2009.

4. Composition as in any claim hereinbefore, **characterized in that** it comprises inulin.

5. Composition as in claim 4, **characterized in that** the inulin is present between 35% and 70% by weight of the total weight of the composition.

6. Composition as in any claim hereinbefore, **characterized in that** it comprises corn starch as a thickener.

7. Composition as in claim 6, **characterized in that** the corn starch is present between 2% and 12% by weight of the total weight of the composition.

8. Composition as in any claim hereinbefore, **characterized in that** it comprises magnesium stearate as a lubricant.

9. Composition as in claim 8, **characterized in that** the magnesium stearate is present between 0.1% and 5% by weight of the total weight of the composition.

10. Formulation for vaginal use **characterized in that** it comprises a composition as in any claim from 1 to 9, **and in that** tindalized *Lactobacillus casei* is present at a concentration between 5% and 15% by weight of the total weight of the formulation, and tindalized *Streptococcus thermophilus* is present at a concentration between 5% and 15% by weight of the total weight of the formulation.

11. Formulation as in claim 10, **characterized in that** it is produced in a choice of the following forms: hard capsule, softgel, liquid, solution, biphasic liquid system, suspension, semi-solid form, such as gel, cream or foam, or solid form, such as powder, granules, tablets and equivalent forms.

12. Method for the preparation of a composition for the treatment of the vaginal mucosa, **characterized in that** it comprises associating tindalized *Lactobacillus casei* between 5% and 15% by weight of the total weight of the composition, tindalized *Streptococcus thermophilus* between 5% and 15% in weight of the total weight of the composition and hyaluronic acid.

**Patentansprüche**

1. Zusammensetzung zur Behandlung der Vaginalschleimhaut, **dadurch gekennzeichnet, dass** sie tindalisierte *Lactobacillus casei* zwischen 5 Gew.-% und 15 Gew.-% des Gesamtgewichts der Zusammensetzung und tindalisierte *Streptococcus thermophilus* zwischen 5 Gew.-% und 15 Gew.-% des Gesamtgewichts der Zusammensetzung aufweist, **und dass** sie Hyaluronsäure aufweist.

2. Zusammensetzung nach Anspruch 1, **dadurch gekennzeichnet, dass** die Hyaluronsäure zwischen 4 Gew.-% und 14 Gew.-% des Gesamtgewichts der Zusammensetzung vorhanden ist.

3. Zusammensetzung nach Anspruch 1 oder 2, **dadurch gekennzeichnet, dass** die Hyaluronsäure ein Molekulargewicht zwischen 50 und 1800 kDa aufweist, wobei das Molekulargewicht gemessen wird mittels der Formel MW = $([\eta] x 10^5/36)^{1/0.078}$, wobei $[\eta]$ die intrinsische Viskosität der Hyaluronsäure in $m^3/kg$ ist, gemessen mittels der in der Europäischen Pharmakopöe (Ph. Eur.), Monographie Nr. 1472, 1/2009, definierten Methode.

4. Zusammensetzung nach einem der vorstehenden Ansprüche, **dadurch gekennzeichnet, dass** sie Inulin aufweist.

**5.** Zusammensetzung nach Anspruch 4, **dadurch gekennzeichnet, dass** das Inulin zwischen 35 Gew.-% und 70 Gew.-% des Gesamtgewichts der Zusammensetzung vorhanden ist.

**6.** Zusammensetzung nach einem der vorstehenden Ansprüche, **dadurch gekennzeichnet, dass** sie Maisstärke als Verdickungsmittel aufweist.

**7.** Zusammensetzung nach Anspruch 6, **dadurch gekennzeichnet, dass** die Maisstärke zwischen 2 Gew.-% und 12 Gew.-% des Gesamtgewichts der Zusammensetzung vorhanden ist.

**8.** Zusammensetzung nach einem der vorstehenden Ansprüche, **dadurch gekennzeichnet, dass** sie Magnesiumstearat als Gleitmittel aufweist.

**9.** Zusammensetzung nach Anspruch 8, **dadurch gekennzeichnet, dass** das Magnesiumstearat zwischen 0.1 Gew.-% und 5 Gew.-% des Gesamtgewichts der Zusammensetzung vorhanden ist.

**10.** Formulierung zur vaginalen Anwendung, **dadurch gekennzeichnet, dass** sie eine Zusammensetzung nach einem der Ansprüche 1 bis 9 aufweist, **und dass** tindalisierter *Lactobacillus casei* in einer Konzentration zwischen 5 Gew.-% und 15 Gew.-% des Gesamtgewichts der Formulierung und tindalisierter *Streptococcus thermophilus* in einer Konzentration zwischen 5 Gew.-% und 15 Gew.-% des Gesamtgewichts der Formulierung vorhanden ist.

**11.** Formulierung nach Anspruch 10, **dadurch gekennzeichnet, dass** sie in einer Auswahl in einer der folgenden Formen hergestellt wird: Hartkapsel, Weichkapsel, Flüssigkeit, Lösung, zweiphasiges Flüssigkeitssystem, Suspension, halbfeste Form, wie Gel, Creme oder Schaum, oder feste Form, wie Pulver, Granulat, Tabletten und gleichwertige Formen.

**12.** Verfahren zur Herstellung einer Zusammensetzung zur Behandlung der Vaginalschleimhaut, **dadurch gekennzeichnet, dass** es die Kombination von tindalisierten *Lactobacillus casei* zwischen 5 Gew.-% und 15 Gew.-% des Gesamtgewichts der Zusammensetzung, tindalisierten *Streptococcus thermophilus* zwischen 5 Gew.-% und 15 Gew.-% des Gesamtgewichts der Zusammensetzung und von Hyaluronsäure aufweist.

## Revendications

**1.** Composition pour le traitement de la muqueuse vaginale, **caractérisée en ce qu'**elle comprend *Lactobacillus casei* tyndallisé entre 5% et 15% en poids du poids total de la composition, et *Streptococcus thermophilus* tyndallisé entre 5% et 15% en poids du poids total de la composition, **et en ce qu'**elle comprend de l'acide hyaluronique.

**2.** Composition selon la revendication 1, **caractérisée en ce que** l'acide hyaluronique est présent entre 4% et 14% en poids du poids total de la composition.

**3.** Composition selon la revendication 1 ou 2, **caractérisée en ce que** l'acide hyaluronique présente une masse moléculaire comprise entre 50 et 1800 kDa, ladite masse moléculaire étant mesurée au moyen de la formule MW = $([\eta]\times 10^5/36)^{1/0.78}$, dans laquelle $[\eta]$ est la viscosité intrinsèque de l'acide hyaluronique en $m^3/kg$ mesurée au moyen du procédé défini dans la Pharmacopée européenne (Ph. Eur.), monographie n. 1472, 1/2009.

**4.** Composition selon une quelconque revendication précédente, **caractérisée en ce qu'**elle comprend de l'inuline.

**5.** Composition selon la revendication 4, **caractérisée en ce que** l'inuline est présente entre 35% et 70% en poids du poids total de la composition.

**6.** Composition selon une quelconque revendication précédente, **caractérisée en ce qu'**elle comprend de l'amidon de maïs en tant qu'épaississant.

**7.** Composition selon la revendication 6, **caractérisée en ce que** l'amidon de maïs est présent entre 2% et 12% en poids du poids total de la composition.

**8.** Composition selon une quelconque revendication précédente, **caractérisée en ce qu'**elle comprend du stéarate de magnésium en tant que lubrifiant.

9. Composition selon la revendication 8, **caractérisée en ce que** le stéarate de magnésium est présent entre 0.1% et 5% en poids du poids total de la composition.

10. **Formulation à** usage vaginal **caractérisée en ce qu'**elle comprend une composition selon l'une quelconque des revendications 1 à 9, **et en ce que** *Lactobacillus casei* tyndallisé est présent en une concentration comprise entre 5% et 15% en poids du poids total de la formulation, et *Streptococcus thermophilus* tyndallisé est présent en une concentration comprise entre 5% et 15% en poids du poids total de la formulation.

11. Formulation selon la revendication 10, **caractérisée en ce qu'**elle est produite sous l'une des formes suivantes : une gélule, une gélule molle, un liquide, une solution, un système liquide biphasique, une suspension, une forme semi-solide, telle qu'un gel, une crème ou une mousse, ou une forme solide, telle qu'une poudre, des granulés, des comprimés et des formes équivalentes.

12. Procédé de préparation d'une composition pour le traitement de la muqueuse vaginale, **caractérisé en ce qu'**il comprend l'association de *Lactobacillus casei* tyndallisé entre 5% et 15% en poids du poids total de la composition, de *Streptococcus thermophilus* tyndallisé entre 5% et 15% en poids du poids total de la composition et d'acide hyaluronique.

The absorbance measured at 450 nm is directly proportional to the quantity of interleukin produced by the tissues. The values are expressed as averages ± standard deviation derived from the results of the treatment of at least 3 tissues. The statistical processing of the data was carried out by means of the Student's t-test. Values of $p < 0.05$ are considered significant. NC = non-treated tissues; LPS = tissues treated with lipopolysaccharide to stimulate the production of IL-1α; TS+LPS = tissues treated with LPS and with the tested sample to verify its soothing action. ** $p < 0.01$ vs LPS

fig. 1

The absorbance measured at 540 nm is proportional to cell vitality. The percentages have been calculated based on the absorbance values at 540 nm and considering the absorbance of the negative control (non-treated cells) as 100%. The statistical processing of the data was carried out by means of the Student's t-test. Values of $p < 0.05$ are considered significant. NC = non treated tissues; LPS = tissues treated with lipopolysaccharide to stimulate the production of IL-α; TS+LPS = tissues treated with LPS and with the tested sample to verify its soothing action. ** $p < 0.01$ vs LPS

fig. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113662998

**Non-patent literature cited in the description**

- **GUARALDI et al.** Tyndallized lactic ferments: new possible therapies in treating vaginitis. *Minerva Obstetrics and Gynecology*, 2017, vol. 69, 1